# EUROPEAN PATENT APPLICATION

(11) **EP 2 174 651 A1**
(43) Date of publication of application: **14.04.2010**
(21) Application number: 08017731.4
(22) Date of filing: 09.10.2008
(51) Int. Cl.: A61K 9/06, A61K 47/02, A61K 31/704, A61P 35/00

(54) **A polymer-free hydrogel**

(71) Applicant: Charité-Universitätsmedizin Berlin (Charité), 10117 Berlin (DE)
(72) Inventor: Meyer, Wolfdietrich, 10117 Berlin (DE); Schellenberger, Eyk, 10117 Berlin (DE); Taupitz, Matthias, 10117 Berlin (DE); Schnorr, Jörg, 10117 Berlin (DE)
(74) Representative: Engelhard, Markus

(57) **Abstract**

The present invention relates to a polymer-free hydrogel of a doxorubicin compound, to a method of producing such hydrogel, to a medical device comprising the hydrogel and to a method of applying the hydrogel to a medical device.

## Description

The present invention relates to a polymer-free hydrogel of a doxorubicin compound, to a method of producing such hydrogel, to a medical device comprising the hydrogel and to a method of applying the hydrogel to a medical device.

Doxorubicin is an anthracyclin glycoside antineoplastic antibiotic produced by Streptomyces sp. It is a topoisomerase II inhibitor and is used in the treatment of a number of neoplastic diseases. It has been used for the treatment of advanced AIDS-related Kaposi's sarcoma that has progressed despite prior combination chemotherapy or in patients who are intolerant of such combination therapy. Moreover doxorubicin hydrochloride is indicated for the treatment of metastatic ovarial carcinoma that is refractory to both paclitaxel- and platinum based chemotherapy regimens. Another indication is metastatic breast carcinoma.

A number of delivery forms and drug administration dosage units have been devised. Doxorubicin hydrochloride has been encapsulated in liposome formulations which are injected into a patient. A slow release of the active ingredient is achieved and doxorubicin hydrochloride has a longer halflife in a patient's body. Another form of a controlled release doxorubicin containing composition is based on polyethylene glycol (PEG) gels and/or dextran-methacrylate gels. Such compositions provide a rapid initial release of doxorubicin over a period of several hours followed by a slow release.

None of the aforementioned formulations are particularly useful for coating medical devices, and patients' compliance with some of these formulations may be low. Accordingly, it was an object of the present invention to provide for alternative formulations of doxorubicin.

The objects of the present invention are solved by a polymer-free, lipid-free hydrogel consisting of

The objects of the present invention are solved by a polymer-free, lipid-free hydrogel consisting of
a) a doxorubicin compound selected from doxorubicin base, and doxorubicin acid addition salts, such as doxorubicin hydrochloride,
b) a salt, and
c) water.

It should be noted that the "salt" of b) is not identical with the doxorubicin acid addition salt of a).

In one embodiment, the salt is selected from salts having metal ions in it, preferably mono, di and/or trivalent metal ions, more preferably NaCl and KCI and combinations of the foregoing.

In one embodiment, the concentration of salt is in the range of from 0.1 µM to saturation-concentration of said salt, and wherein the concentration of doxorubicin compound is in the range of from 0.1 mM to saturation-concentration of said doxorubicin compound.

In one embodiment, the hydrogel according to the present invention has a pH in the range of from 0 to 9.

In one embodiment, the hydrogel according to the present invention is produced by a method comprising the steps:
i) preparing a mixture of said doxorubicin compound and water and dissolving said doxorubicin compound, optionally by addition of a base, or
i') preparing a mixture of said doxorubicin compound and water containing a base,
ii) adjusting the pH to a range from 0-9, preferably from 6 to 8, more preferably from 6.8 to 7.6, by addition of an acid or a base,
iii) adjusting the temperature of the solution resulting from step ii) to a temperature which is the desired melting point of said gel and which is in the range from 0°C to 70°C,
iv) and optionally, adding a salt thereto until gelation starts, and then discontinuing addition of salt and, further optionally, adjusting the temperature of said gelating solution to a temperature below said melting point, preferably a temperature in the range < 30°C.

In one embodiment, said base is selected from mineral bases, preferably NaOH or KOH, and wherein said acid is selected from mineral acids preferably HCI, H₂SO₄ H₃PO₄..

In one embodiment, the hydrogel according to the present invention is thixotropic.

In one embodiment, the hydrogel according to the present invention consists of
a) a doxorubicin compound at a concentration in the range of from 0.1 mM to saturation-concentration of the doxorubicin compound,
b) salt at a concentration in the range of from 0.1 µM to saturation-concentration of the salt, and
c) water.

In one embodiment, the hydrogel according to the present invention has a melting point from 0°C to 70°C.

The objects of the present invention are also solved by the hydrogel according to the present invention for use as a pharmaceutical.

The objects of the present invention are also solved by the hydrogel according to the present invention for the treatment of cancerous diseases, in particular cervical carcinoma, leukemias, Hodgkin's lymphoma, cancers of the bladder, stomach, lung, ovaries, thyroid, soft tissue sarcoma, multiple myeloma, mammary carcinoma and Kaposi sarcoma.

In one embodiment, said hydrogel is administered to a patient in need thereof by injection.

The objects of the present invention are also solved by a medical device comprising the hydrogel according to the present invention.

In one embodiment, the hydrogel is arranged in a layer on or in said medical device.

In one embodiment, the medical device is selected from a syringe, a catheter, a stent, an indwelling prosthetic, a porous implant for drug depot, or a drug carrier device.

The objects of the present invention are also solved by a method of producing the hydrogel according to the present invention comprising the steps:
i) preparing a mixture of a doxorubicin compound, as defined above, and water, and dissolving said doxorubicin compound, optionally by addition of a base, or
i') preparing a mixture of said doxorubicin compound and water containing a base,
ii) adjusting the pH to a range from 0-9, preferably from 6 to 8, more preferably from 6.8 to 7.6, by addition of an acid or base,
iii) adjusting the temperature of the solution resulting from step ii) to a temperature which is the desired melting point of said hydrogel and which is in the range from 0°C to 70°C,
iv) and, optionally, adding a salt thereto until gelation starts, and then discontinuing addition of salt and, further optionally, adjusting the temperature of said gelating solution to a temperature below said melting point, preferably a temperature in the range < 30°C.

In one embodiment, said base is selected from mineralic bases, preferably NaOH or KOH, and wherein said acid is selected from mineralic acids, preferably HCI or H₂SO₄.

In one embodiment, in step iii) said temperature of said solution is adjusted to a temperature in the range from > 30°C to 70°C, and wherein, in step (iv) the temperature is adjusted to a range < 30°C.

It should be noted that the presence of the salt in said hydrogel may be achieved by the addition of acid or base or both together, such as HCI and/or NaOH, or by the addition of salt itself, such as NaCI, KCl. Alternatively, salt may also already be present in the commercially available preparation of doxorubicin compound. For example one possibility of preparing a hydrogel having doxorubicin hydrochloride as the doxorubicin compound comprises the steps:
- preparing a mixture of said doxorubicin hydrochloride and water, with doxorubicin hydrochloride being preferably present at a concentration above the saturation concentration of said doxorubicin hydrochloride in water, adding base, such as NaOH to dissolve said doxorubicin hydrochloride, adjusting the pH to a pH in the range from 6 to 8, using acid, e.g. HCI, and, optionally, mechanically agitating said mixture, for example by shaking or stirring, until gelation starts. In this procedure, the presence of salt (in this case NaCl) is achieved by addition of both acid and base. In other embodiments, the presence of salt may be achieved by addition of salt as a solid or salt solution. The two aforementioned possibilities of achieving the presence of salt may, of course, also occur in a combined fashion.

The objects of the present invention are also solved by a method of applying a hydrogel according to the present invention to a medical device, by dipping said medical device in said hydrogel, immersing said medical device in said hydrogel, spraying said hydrogel on said medical device, pouring said hydrogel on said medical device, or taking said hydrogel up in said medical device.

In one embodiment, said medical device has a surface temperature below the melting point of said hydrogel, and said hydrogel, prior to being applied to said medical device, has a temperature above the melting point of said hydrogel.

The inventors have surprisingly found that doxorubicin itself can form a hydrogel without the need to recur to polymer-based gels or liposome-based formulations. If one dissolves a doxorubicin compound, such as doxorubicin hydrochloride in water in the presence of a salt, a hydrogel is formed.

Without wishing to be bound by any theory, the present inventors believe that the formation of the gel is presumably due to the so called π-stacking between a foreign ion and the aromatic π-system in doxorubicin and or the supramolecular interaction beween the additive ions, water and the functional groups that make doxorubicin watersoluble.

By choosing the right additives and synthesis condition it is possible to obtain a thixotropic gel with a defined melting temperature. An embodiment of a procedure to obtain a hydrogel with defined melting point is as follows: First, a doxorubicin compound is dissolved in water, optionally by addition of a base. Thereafter, the pH is adjusted to a range from 0-9, preferably from 6-8, more preferably from 6.8 to 7.6, by addition of an acid or a base. Generally speaking, more doxorubicin can be dissolved at higher pH values, since the saturation concentration of doxorubicin compound is pH dependent. The resulting solution is then adjusted to the desired melting point temperature ("melting point") of the gel which desired melting point temperature may lie in the range of from 0°C to 70°C. Thereafter, a salt may be added, either continuously or in a stepwise fashion. The addition may be as a solid or as an aqueous solution. Subsequently gelation will start, and the addition of the salt is discontinued. The mixture is subsequently adjusted to a temperature below the melting point of the gel, preferably < 30°C, more preferably in a range of 1 °C to 29.9°C. Again, it should be noted, that the aforementioned addition of salt is optional. In those embodiments wherein, e.g. through the addition of base or acid or both for pH adjustment, or through the presence of salt in the doxorubicin compound preparation, salt is already present, no extra addition of salt will be necessary.

The gels thus formed does not contain additional ingredients except for water and salt, and therefore patient compliance is likely to be much better. The gels thus formed are thixotropic and therefore are particularly amenable to administration by injection. On the other hand, they can also be easily applied to medical devices, such as catheters, stents, indwelling prosthetics, porous implants for drug depots, other drug carrier devices on which they form a layer. Such layers may be applied by any conventional technique such as dipping, immersing, spraying etc. Because the gel is thixotropic it is particularly suitable for processing. By choosing the salt content and the content of the doxorubicin compound appropriately, the melting point can be adjusted to lie in a range of from 0°C-70°C. As used herein, the term "melting point", when used in the context of a hydrogel is meant to refer to the temperature above which the gel turns into a liquid and below which the gel remains in the gel state.

The gel in accordance with the present invention may be injected into tumour tissue and may act as a store for active ingredients providing for a slow release thereof. There are a number of doxorubicin compounds suitable in accordance with the present invention, such as doxorubicin free base, doxorubicin acid addition salts, such as doxorubicin hydrochloride.

As used herein, the term "saturation concentration of a salt/doxorubicin compound" is meant to refer to the maximum concentration of the salt/doxorubicin compound which, under the existing conditions, may get dissolved in the respective solvent. Such saturation concentration may depend on a number of variables, including temperature, solvent, other solutes and pH, and can be easily determined by someone skilled in the art.

The term "thixotropic", as used herein in the context of a gel, refers to a gel that appears to be a gel-like solid and maintains a defined shape of its own until it is subjected to a shearing force or some other disturbance, such as shaking, in which case it then fluidifies and acts as a sol or liquid and flows more freely. Such thixotropic behaviour is reversible. The aforementioned sol, when allowed to stand undisturbed, reverts to the gel again. Typical thixotropic gels include oil well drilling mud, specific paints and printing inks and clays.

The melting point of the hydrogel in accordance with the present invention is important for example with respect to the coating or covering of medical devices. For example, by dipping a device, which has a temperature below the melting point of the hydrogel, into a solution just over the melting point of the hydrogel will result in a defined coverage of doxorubicin gel on such device. Another example is the application of a gel in accordance with the present invention that has a melting point just below the body temperature, to or into a body. Because such gel is thixotropic, it can be directly injected by means of a syringe, and within the body, the doxorubicin is released from the gel because the gel has turned into a liquid due to its being adjusted to a temperature above its melting point.

In the following, reference is made to the figures, wherein
Figure 1 shows a batch of RKO-cells grown in the absence of a thixotropic doxorubicin hydrochloride gel,
Figure 2 shows RKO-cells grown for 24 h in the presence of a polypropylene surface (1 cm²) covered with a doxorubicin hydrochloride gel, and
Figure 3 shows RKO-cells grown for 24 h in the presence of a polypropylene surface (2 cm²) covered with a doxorubicin hydrochloride gel.

Moreover, reference is made to the following example which is given to illustrate, not to limit the present invention.

### Example

### i) Preparation of a polymer-free, lipid-free doxorubicin hydrogel.

47 mg (8.10 x 10⁻⁵ mol) doxorubicin hydrochloride was added to 1 ml water. A saturated solution containing a sediment of undissolved doxorubicin hydrochloride was obtained. After addition of 0.5 equivalents NaOH (40 µl of a 1 M NaOH solution) the sediment dissolved completely, and the pH was adjusted to 7 using 1 M HCI. A red transparent gel formed having a density of ca. 1 g/ml, a melting point of approximately 40-41°C and a concentration of active ingredient of 80 mM. The gel was thixotropic.

### ii) Coating of a surface using a thixotropic doxorubicin hydrochloride gel.

Two sterile polypropylene surfaces (PPO) were immersed into a thixotropic doxorubicin hydrochloride gel, as prepared in i) (see above) (0.29 M doxorubicin hydrochloride), and a) 4 mg of the gel were coated on 1 cm² polypropylene surface and b) 7.6 mg of the gel were coated on 2 cm² polypropylene surface.

### iii) Growth of the cells in the presence of doxorubicin hydrochloride

Three batches of RKO-cells (colon carcinoma, human) were grown in medium in the presence of antibiotics (streptomycin) for 24 h and the presence or absence of a thixotropic doxorubicin hydrochloride gel in accordance with the present invention. The three batches were treated
1. without a thixotropic doxorubicin hydrochloride gel
2. in the presence of the polypropylene surface of a) (i.e. coated with a thixotropic doxorubicin hydrochloride gel, see ii),
3. in the presence of a polypropylene surface b) (i.e. coated with a thixotropic doxorubicin hydrochloride gel, see ii).

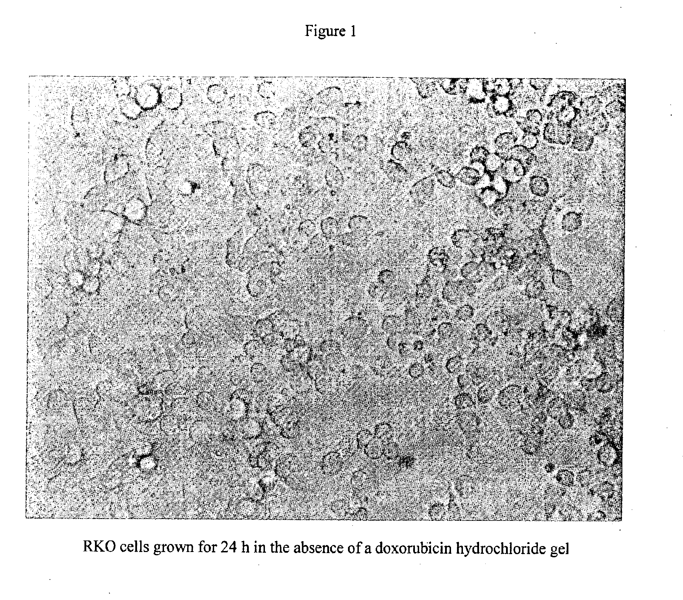

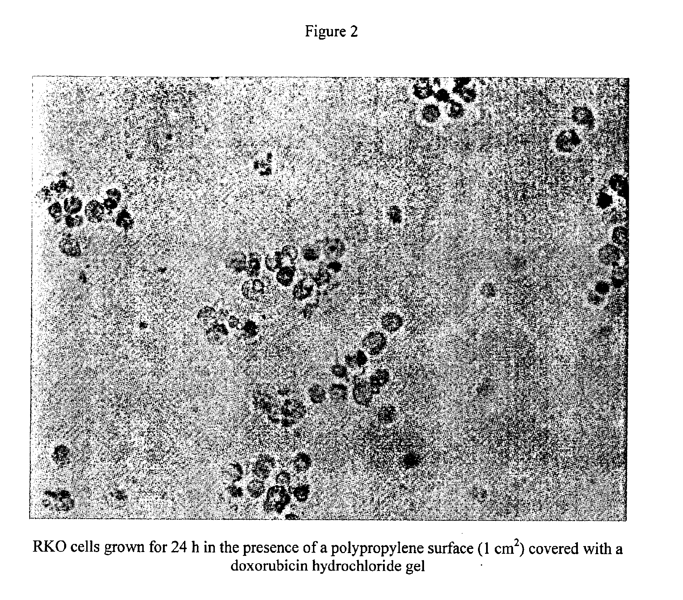

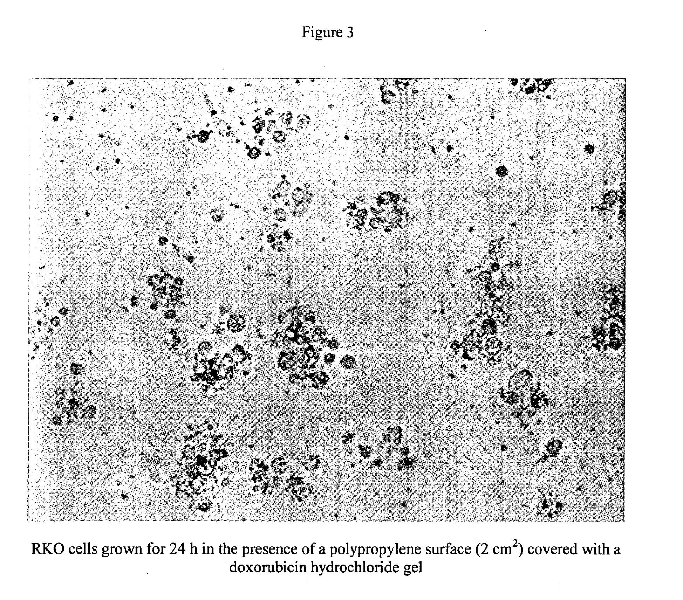

The untreated cancer cells multiplied numerously and had a characteristic appearance in that they formed an adherent cell lawn having a high contact number to adjacent cells, and involving the formation of cell extensions (see also figure 1). The cells which had been grown in the presence of the thixotropic doxorubicin hydrochloride gel multiplied less intensely and had a pathological appearance in that they formed round-shaped free floating cells without cell extensions (see figures 2 and 3).

These results suggest that the hydrogel in accordance with the present invention may be particularly useful in the treatment of neoplastic diseases. The hydrogel is particularly versatile in that it is a thixotropic gel. Moreover since it only contains the active ingredient and salts, such as NaCI and/or KCl, patient compliance is good.

The features of the present invention disclosed in the specification, the claims and/or in the accompanying drawings, may, both separately, and in any combination thereof, be material for realising the invention in various forms thereof.

## Claims

1. A polymer-free, lipid-free hydrogel consisting of
a) a doxorubicin compound selected from doxorubicin base, and doxorubicin acid addition salts, such as doxorubicin hydrochloride,
b) a salt, and
c) water.

2. The hydrogel according to claim 1, wherein the salt is selected from salts having metal ions in it, preferably mono, di and/or trivalent metal ions, more preferably NaCI and KCI and combinations of the foregoing.

3. The hydrogel according to any of claims 1-2, wherein the concentration of salt is in the range of from 0.1 µM to saturation-concentration of said salt, and wherein the concentration of doxorubicin compound is in the range of from 0.1 mM to saturation-concentration of said doxorubicin compound.

4. The hydrogel according to any of claims 1-3, having a pH in the range of from 0 to 9.

5. The hydrogel according to any of claims 1 - 4, produced by a method comprising the steps:
i) preparing a mixture of said doxorubicin compound and water and dissolving said doxorubicin compound, optionally by addition of a base, or
i') preparing a mixture of said doxorubicin compound and water containing a base,
ii) adjusting the pH to a range from 0-9, preferably from 6 to 8, more preferably from 6.8 to 7.6, by addition of an acid or a base,
iii) adjusting the temperature of the solution resulting from step ii) to a temperature , which is the desired melting point of said hydrogel and which is in the range from 0°C to 70°C,
iv) and, optionally, adding a salt thereto until gelation starts, and then discontinuing addition of salt and, further optionally, adjusting the temperature of said gelating solution to a temperature below said melting point, preferably a temperature in the range < 30°C.

6. The hydrogel according to claim 5, wherein said base is selected from mineral bases, preferably NaOH or KOH, and wherein said acid is selected from mineral acids preferably HCI, H₂SO₄, H₃PO₄..

7. The hydrogel according to any of the foregoing claims, which is thixotropic.

8. The hydrogel according to any of the foregoing claims, consisting of
a) a doxorubicin compound at a concentration in the range of from 0.1 mM to saturation-concentration of the doxorubicin compound,
b) salt at a concentration in the range of from 0.1 µM to saturation-concentration of the salt, and
c) water.

9. The hydrogel according to any of the foregoing claims, having a melting point from 0°C to 70°C.

10. The hydrogel according to any of the foregoing claims for use as a pharmaceutical.

11. The hydrogel according to any of claims 1 - 9, for the treatment of cancerous diseases, in particular cervical carcinoma, leukemias, Hodgkin's lymphoma, cancers of the bladder, stomach, lung, ovaries, thyroid, soft tissue sarcoma, multiple myeloma, mammary carcinoma and Kaposi sarcoma.

12. The hydrogel according to any of claims 10-11, wherein said hydrogel is administered to a patient in need thereof by injection.

13. A medical device comprising the hydrogel according to any of claims 1-9.

14. The medical device according to claim 13, wherein the hydrogel is arranged in a layer on or in said medical device.

15. The medical device according to claim 14, wherein the medical device is selected from a syringe, a catheter, a stent, an indwelling prosthetic, a porous implant for drug depot, or a drug carrier device.

16. A method of producing the hydrogel according to any of claims 1 - 9 comprising the steps:
i) preparing a mixture of a doxorubicin compound, as defined in any of claims 1-9, and water, and dissolving said doxorubicin compound, optionally by addition of a base, or
i') preparing a mixture of said doxorubicin compound and water containing a base,
ii) adjusting the pH to a range from 0 - 9, preferably from 6 to 8, more preferably from 6.8 to 7.6, by addition of an acid or base,
iii) adjusting the temperature of the solution resulting from step ii) to a temperature which is the desired melting point of said hydrogel and which is in the range from 0°C to 70°C,
iv) and, optionally, adding a salt thereto until gelation starts, and then discontinuing addition of salt and, further optionally, adjusting the temperature of said gelating solution to a temperature below said melting point, preferably a temperature in the range < 30°C.

17. The method according to claim 16, wherein said base is selected from mineralic bases, preferably NaOH or KOH, and wherein said acid is selected from mineralic acids, preferably HCl or H₂SO₄.

18. The method according to claim 17, wherein in step iii) said temperature of said solution is adjusted to a temperature in the range from > 30°C to 70°C, and wherein, in step (iv) the temperature is adjusted to a range < 30°C.

19. A method of applying a hydrogel according to any of claims 1 - 9, to a medical device, by dipping said medical device in said hydrogel, immersing said medical device in said hydrogel, spraying said hydrogel on said medical device, pouring said hydrogel on said medical device, or taking said hydrogel up in said medical device.

20. The method according to claim 19, wherein said medical device has a surface temperature below the melting point of said hydrogel, and said hydrogel, prior to being applied to said medical device, has a temperature above the melting point of said hydrogel.
